(19) Europäisches Patentamt
European Patent Office
Office européen des brevets

(11) **EP 2 552 310 B1**

(12) **EUROPÄISCHE PATENTSCHRIFT**

(45) Veröffentlichungstag und Bekanntmachung des Hinweises auf die Patenterteilung:
**23.05.2018 Patentblatt 2018/21**

(51) Int Cl.:
*A61B 5/05* (2006.01)     *A61B 5/055* (2006.01)
*G01R 33/12* (2006.01)     *G01R 33/20* (2006.01)

(21) Anmeldenummer: **11716372.5**

(22) Anmeldetag: **01.04.2011**

(86) Internationale Anmeldenummer:
**PCT/EP2011/001651**

(87) Internationale Veröffentlichungsnummer:
**WO 2011/120713 (06.10.2011 Gazette 2011/40)**

(54) **VERFAHREN ZUR BILDGEBUNG MITTELS MAGNETISCHER KLEINSTPARTIKEL SOWIE VORRICHTUNG HIERFÜR**

IMAGING METHOD USING MAGNETIC SMALL PARTICLES, AND CORRESPONDING DEVICE

PROCÉDÉ D'IMAGERIE PAR DE PETITES PARTICULES MAGNÉTIQUES ET DISPOSITIF UTILISÉ À CET EFFET

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **01.04.2010 DE 102010013900**

(43) Veröffentlichungstag der Anmeldung:
**06.02.2013 Patentblatt 2013/06**

(73) Patentinhaber:
• **Rückert, Martin**
**97234 Reichenberg (DE)**
• **Behr, Volker C.**
**97218 Gerbrunn (DE)**

(72) Erfinder:
• **Rückert, Martin**
**97234 Reichenberg (DE)**
• **Behr, Volker C.**
**97218 Gerbrunn (DE)**

(74) Vertreter: **FDST Patentanwälte**
**Nordostpark 16**
**90411 Nürnberg (DE)**

(56) Entgegenhaltungen:
WO-A1-2009/037636    WO-A1-2010/041178
WO-A2-2004/091386    WO-A2-2004/091390
WO-A2-2004/091408

EP 2 552 310 B1

**Beschreibung**

Die Erfindung betrifft ein neuartiges Verfahren zur Bildgebung mittels magnetischer Kleinstpartikel. Weiterhin betrifft die Erfindung eine zur Durchführung dieses Verfahrens geeignete Vorrichtung.

Ein Verfahren der eingangs genannten Art, auch "Magnetic-Particle-Imaging" genannt, sowie eine zur Durchführung geeignete Vorrichtung sind aus dem Artikel "Tomographic Imaging Using the Non-Linear Response of Magnetic Particles", Bernhard Gleich, Jürgen Weizenecker, Nature, Vol. 435, 30. Juni 2005 bekannt. Dort wird der nicht-lineare Zusammenhang zwischen der Magnetisierung eines ferromagnetischen Kleinstpartikels bezüglich eines äußeren magnetischen Feldes zu einer räumlichen Bildgebung der gegebenen Verteilung der Kleinstpartikel verwendet. Dazu wird insbesondere ausgenutzt, dass die Magnetisierung bei starken Magnetfeldern einen Sättigungswert erreicht, während in einem Bereich um den Nullpunkt der Feldstärke eine in etwa lineare Abhängigkeit von der Feldstärke gegeben ist. Wird das äußere Magnetfeld um den Nullpunkt sinusförmig variiert, so ergibt sich aufgrund der Sättigungseffekte als Antwortfunktion der Magnetisierung eine Rechteckfunktion. In den Frequenzraum transformiert setzt sich das Magnetisierungssignal damit aus einer Summe der Harmonischen der Grundfrequenz des eingestrahlten Magnetfeldes zusammen. Das beispielsweise induktiv abgegriffene Signal der Magnetisierung kann infolge dessen leicht von dem eingestrahlten Erregersignal getrennt werden, indem der Grundfrequenzanteil absepariert wird.

Zur Ortskodierung wird im Untersuchungsbereich ein starkes magnetisches Gradientenfeld mit einem feldfreien Punkt am Messvolumen erzeugt. Da die Magnetisierung der Kleinstpartikel außerhalb des feldfreien Punktes ihren Sättigungswert erreicht, tragen effektiv nur diejenigen Kleinstpartikel zur Bildgebung bei, die sich am feldfreien Punkt befinden. Durch eine Verschiebung des feldfreien Punktes über den Untersuchungsbereich kann die Anzahl der dort jeweils befindlichen magnetischen Kleinstpartikel über die Amplitude des empfangenen Magnetisierungssignals ermittelt werden. Der feldfreie Punkt kann hierbei entweder durch eine tatsächliche Bewegung der Messapparatur oder durch eine entsprechende Ansteuerung des Gradientenfeldes, auch Selektionsfeld genannt, bewegt werden.

Durch das bekannte Verfahren zur Bildgebung mittels magnetischer Kleinstpartikel kann sowohl in vitro als auch in vivo eine Verteilung der magnetischen Kleinstpartikel ortsaufgelöst beobachtet werden. Durch die Verwendung eines magnetischen Kleinstpartikel enthaltenden Kontrastmittels können insofern räumliche Aufnahmen von Geweben, Organen oder eines Gefäßsystems erhalten werden. Auch können Stoffwechselprozesse beobachtet werden, indem die magnetischen Kleinstpartikel beispielsweise spezifischen an Stoffwechselprozessen teilnehmenden organischen Makromolekülen angeheftet werden. Ebenso ist es möglich, die magnetischen Kleinstpartikel mit funktionalen Gruppen zu versehen, die an spezifische Körperzellen ankoppeln, so dass aus deren Verteilung Rückschlüsse geschlossen werden können. Über die zeitliche Auflösung der Verteilung des Kontrastmittels können auch dynamische Prozesse wie z.B. der Blutfluss durch ein Organ etc. abgebildet bzw. dargestellt werden.

Der mit dem bekannten Verfahren erzielbaren räumlichen Auflösung sind durch den notwendigen Gradienten des Selektionsfeldes Grenzen gesetzt. Die räumliche Auflösung wird umso besser, je größer der Gradient des Selektionsfeldes gewählt wird. Für magnetische Kleinstpartikel von einigen 10 nm Durchmesser werden typischerweise Gradienten um 5 T/m (Tesla pro Meter) und darüber benötigt, um eine räumliche Auflösung im Submillimeter-Bereich zu erzielen. Solche hohen Gradientenfelder lassen sich praktisch jedoch nur mit supraleitenden Spulen oder mittels Neodym-Magneten erzielen, was nachteiligerweise die Kosten für eine derartige Bildgebung deutlich erhöht. Typische aus dem Kernspinresonanz-Verfahren bekannte Gradientenfelder bewegen sich zum Vergleich bei etwa 100 mT/m.

Weiter ist eine höhere räumliche Auflösung des bekannten Verfahrens mit einer Verschlechterung des Signal-zu-Rausch-Verhältnisses verbunden. Je steiler nämlich der Gradient des Selektionsfeldes gewählt ist, desto kleiner ist der Bereich einer linearen Antwort der Magnetisierung der Kleinstpartikel und desto weniger Kleinstpartikel tragen zum Messsignal insgesamt bei.

Dokument WO 2004/091390 A2 offenbart ein Verfahren und eine Vorrichtung für "Magnetic Particle-Imaging", wobei in einer Ausführungsform ein zusätzliches rotierendes Magnetfeld zur gezielten Freisetzung von Wirkstoffen von einem magnetischen Partikel beschrieben wird.

Die Erfindung hat sich die Aufgabe gestellt, ein Verfahren zur Bildgebung mittels magnetischer Kleinstpartikel und eine hierfür geeignete Vorrichtung bereitzustellen, womit sich gegenüber dem Stand der Technik eine verbesserte räumliche Auflösung erzielen lässt und deren Realisierung mit geringeren Kosten verbunden ist.

Diese Aufgabe wird erfindungsgemäß gelöst durch ein Verfahren mit den Merkmalen des Anspruchs 1.

Die Erfindung löst sich dabei überraschend von dem bislang von der Fachwelt eingeschlagenen Weg, zur Bildgebung mittels magnetischer Kleinstpartikel den nicht-linearen Zusammenhang der Magnetisierung von einem äußeren magnetischen Feld zu nutzen. Vielmehr verwendet die Erfindung zur Bildgebung die Tatsache, dass die Rotation der magnetischen Kleinstpartikel oder deren Magnetisierung in einem äußeren rotierenden magnetischen Feld abhängig von Feldstärke und Rotationsfrequenz in einen Bereich synchroner und in einen Bereich asynchroner Drehung zerfällt. Dies liegt im Falle eines mechanisch rotierenden Kleinstpartikels an Reibungstermen gegenüber der Umgebung. Rotiert die Magnetisierung im Kristallgitter des Kleinstpartikels, so sind andere Reibungsterme verantwortlich. Ist das vom äußeren Magnetfeld übertragene Drehmoment gegenüber den Reibungstermen zu gering, so bleibt die Magnetisierung bzw. der Kleinstpartikel gegenüber der Rotation des Magnetfeldes zurück, da es gegenüber der Umgebung verharren möchte. Es entsteht eine nicht-lineare Fluktuation der Drehung der Magnetisierung gegenüber der Rotation des Magnetfeldes. Im Mittel ergibt sich dabei ein messbarer Rotationsdrift.

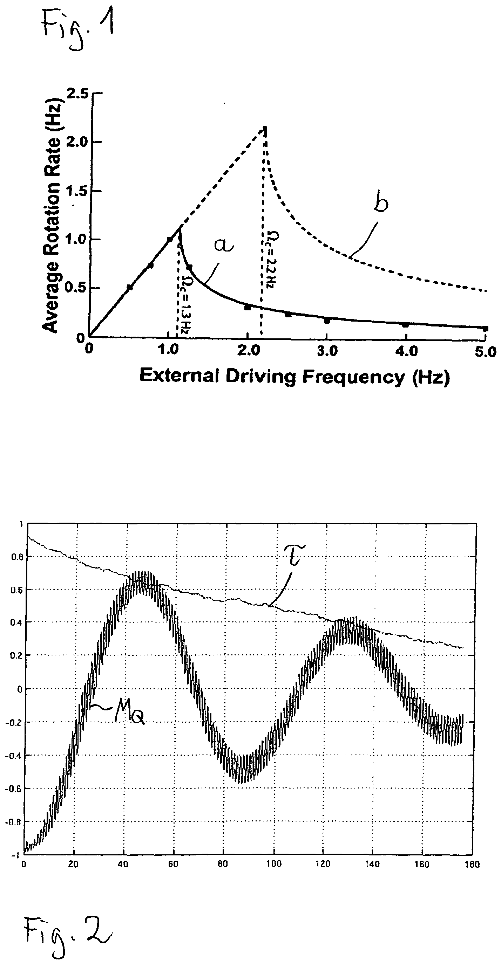

Entsprechend der US 2008/0220411 A1 rotiert die Magnetisierung des Kleinstpartikels abhängig von der Feldstärke des äußeren Magnetfeldes im Mittel bis zu einer kritischen Frequenz $\Omega_c$ synchron zur Rotation des Magnetfeldes. Überschreitet die Rotationsfrequenz des Magnetfeldes die kritische Rotationsfrequenz $Q_c$, so dreht die Magnetisierung des Kleinstpartikels mit einer verringerten mittleren Rotationsfrequenz asynchron. Diese mittlere, asynchrone Drehung der Magnetisierung kann als ein Frequenzterm der Magnetisierung beobachtet werden. Dieser Verlauf der mittleren Rotationsfrequenz der Magnetisierung des Kleinstpartikels wird aus Fig. 1 ersichtlich. Bei kleinerer Feldstärke ergibt sich eine niedrigere kritische mittlere Rotationsfrequenz $\Omega_c$ als bei größerer Feldstärke. Dies lässt sich an den eingezeichneten Kurvenverläufen a und b in Fig. 1 entnehmen. Der Zusammenhang zwischen kritischer mittlerer Rotationsfrequenz $\Omega_c$ und der Feldstärke B des äußeren Magnetfeldes ist dazu gegeben durch:

$$\Omega_c = \frac{mB}{\gamma} \ .$$

Dabei bezeichnet m das magnetische Moment des Kleinstpartikels. Der Term $\gamma$ beschreibt einen Reibungsterm, der im Falle einer mechanischen Rotation des Kleinstpartikels die Viskosität der Umgebung und einen Formfaktor des Kleinstpartikels beinhaltet. Im Falle einer Rotation der Magnetisierung im Kristallgitter des Kleinstpartikels ist der Reibungsterm $\gamma$ empirisch zu ermitteln.

Die Erfindung erkennt nun erstmalig, dass sich die asynchrone Rotation der Magnetisierung der Kleinstpartikel zur Bildgebung nutzen lässt, da in diesem Fall die erfassbare Frequenz der gegenüber dem äußeren Magnetfeld drehenden Magnetisierung von der Frequenz des Erregerfeldes verschieden ist. Insofern kann die mittlere Rotationsfrequenz der Magnetisierung messtechnisch leicht von der Frequenz des Erregerfeldes getrennt werden. In einem Partikelensemble lässt sich der entsprechende Frequenzterm durch Erfassung der superpositionierten Quermagnetisierung (bezüglich der Längsachse, um die das äußere Magnetfeld rotiert) ermitteln. Dies kann beispielsweise induktiv mit einer geeigneten Messspule erfolgen.

Zur Bildgebung und damit zu einer räumlichen Auflösung wird weiter die oben beschriebene Tatsache ausgenutzt, dass die kritische Rotationsfrequenz $\Omega_c$ abhängig von der Feldstärke des äußeren Magnetfeldes ist. Dies ergibt die Möglichkeit, ähnlich dem Kernspinresonanz-Verfahren durch Anlegen eines magnetischen Gradientenfeldes eine Ortskodierung vorzunehmen. Entsprechend Fig. 1 wird sich bei gleicher Rotationsfrequenz des magnetischen Feldes abhängig von der äußeren Feldstärke eine jeweils verschiedene mittlere asynchrone Rotationsfrequenz der Magnetisierung der Kleinstpartikel einstellen. Somit ist der mittleren, asynchronen Rotationsfrequenz der Magnetisierung der Kleinstpartikel eine Ortsinformation aufgeprägt, die zur Bildgebung genutzt wird.

Wird entlang einer vorgegebenen Achse bzw. Raumrichtung ein magnetisches Gradientenfeld erzeugt, so zerfällt die beobachtbare asynchrone Rotation der superpositionierten Quermagnetisierung in Streifen gleicher Frequenzen senkrecht zu dieser Achse. Durch Erfassung der Signalintensitäten, jeweils zugeordnet zu den Frequenzen, wird eine Projektion der Verteilung der superpositionierten Quermagnetisierung auf die Achse des Gradientenfeldes erzeugt. Dies entspricht einer in einer Dimension ortsaufgelösten Bildgebung über die Verteilung der magnetischen Kleinstpartikel bzw. über die Verteilung deren Quermagnetisierung. Das magnetische Gradientenfeld wird somit zu einer Ortskodierung über die sich einstellende Frequenzverteilung genutzt. Diese Möglichkeit wird daher im Folgenden auch als Verfahren der Frequenzkodierung bezeichnet.

Andererseits wird die mittlere, asynchrone Rotationsfrequenz der Magnetisierung der Kleinstpartikel durch die chemische Umgebung beeinflusst. Es entsteht ähnlich dem Kernspinresonanz-Verfahren in den beobachtbaren Rotationsfrequenzen eine sogenannte chemische Verschiebung ("chemical-Shift"). Diese lässt sich durch das angegebene Verfahren ebenfalls sichtbar machen. Hierzu wird das Verfahren mit verschiedenen magnetischen Gradientenfeldern wiederholt, über die die Bereiche unterschiedlicher chemischer Umgebung abgetastet werden. Über die erhaltenen Messinformationen lässt sich ein Bild über die räumliche Verteilung der Umgebungsparameter der Kleinstpartikel erzeugen. Da insbesondere die in den Reibungsterm eingehende Viskosität temperaturempfindlich ist, kann diese Methode beispielsweise zur Sichtbarmachung von Temperaturgradienten, insbesondere bei Stoffwechselvorgängen, etc., verwendet werden.

Es ist auch möglich, über ein für eine vorgegebene Zeitspanne eingeschaltetes Gradientenfeld dem Partikelensemble eine ortsabhängige Phasenlage einzuprägen, und diese Phasenlage zur Bildgebung auszunutzen. Solange das magnetische Gradientenfeld eingeschaltet ist, rotieren nämlich die einzelnen magnetischen Kleinstpartikel ortsabhängig mit jeweils unterschiedlichen asynchronen Drehfrequenzen, woraus sich nach dem Abschalten eine ortsabhängige Phasenlage ergibt, die ebenfalls zur Messung einer ortsaufgelösten Verteilung herangezogen werden kann. Das Gradientenfeld wird vor der eigentlichen Messung zu einer Ortskodierung über die sich einstellende Phasenlage genutzt. Diese Möglichkeit wird im Folgenden als Verfahren der Phasenkodierung bezeichnet.

Gegenüber dem bekannten Kernspinresonanz-Verfahren bietet die vorliegende Erfindung den enormen Vorteil, dass kein statisches Polarisationsfeld zur Ausrichtung erforderlich ist. Vielmehr werden die magnetischen Kleinstpartikel durch das magnetische Rotationsfeld direkt manipuliert. Während zur Ausrichtung der Kernspins Feldstärken von etwa 1,5 T notwendig sind, um ca. 3 ppm der vorhandenen Protonenspins für die Signalerzeugung nutzen zu können, genügen zur Erzeugung einer asynchronen Rotation mit ca. 0.1 % bis nahe 100 % der vorhandenen Magnetisierung der Kleinstpartikel Feldstärken zwischen 0,1 und 100 mT. Während sich das Polarisationsfeld bei der Kernspinresonanz nur mit teuren, supraleitenden Spulen erzeugen lässt, um eine für den klinischen Routineeinsatz taugliche Bildqualität erzielen zu können, kann das magnetische Rotationsfeld zur Sichtbarmachung der rotierenden Magnetisierung der Kleinstpartikel mit günstigen Luftspulen oder wassergekühlten Spulen erzeugt werden.

Beim, Verfahren der Kernspinresonanz kann weiter das Messsignal nur durch Einstrahlen eines hochfrequenten Magnetfeldes mit der Larmor-Frequenz von etwa 60 MHz erzeugt werden. Im Gegensatz hierzu genügt bei dem hier beschriebenen Verfahren zur Erzeugung des Messsignals ein rotierendes Magnetfeld mit einer Frequenz zwischen etwa 1 kHz bis 1 MHz. Im einfachsten Fall ist auch keine separate Gradientenspule zum Erzeugen des Gradientenfeldes notwendig. Vorteilhafterweise kann nämlich das zur Bildgebung erforderliche Gradientenfeld durch entsprechende Ansteuerung der das rotierende Magnetfeld erzeugenden Spulen eingestellt werden.

Zur Erzeugung der hier notwendigen Feldstärken genügen insofern in der Tat simple Luftspulen, die bei Stromstärken von 1 bis 100 A betrieben werden. Derartige Ströme und auch die hier vorliegenden Frequenzen bis etwa 1 MHz lassen sich mit einer üblichen und bekannten Leistungselektronik handhaben. Gegenüber dem Kernspinresonanz-Verfahren ist insofern das hier beschriebene Verfahren eines Magnetic-Particle-Imaging mit enormen Kostenvorteilen verbunden. Diese enormen, auf den großen Unterschieden der notwendigen Feldstärken beruhenden Vorteile sind darin begründet, dass nicht der Kernspin eines Wasserstoffatoms beobachtet, sondern der aus Kopplung der Elektronenspins der Atome im magnetischen Nanopartikel resultierende einzige große Gesamtspin gemessen wird. Das resultierende magnetische Moment in einem etwa 25 nm großen Magnetit-Partikel ist beispielsweise 10-millionenfach höher als das des Wasserstoffprotons. Zwar ist das vorliegende Verfahren gegenüber dem Verfahren der Kernspinresonanz breitbandig. Dieser Nachteil wird jedoch durch die enorm höhere Magnetisierung mehr als ausgeglichen.

Gegenüber dem bislang eingeschlagenen Weg eines Magnetic-Particle-Imaging unter Beobachtung des nicht-linearen Zusammenhangs der Magnetisierung gegenüber einem äußeren, starken Magnetfeld weist das hier beschriebene Verfahren zusätzlich ein wesentlich verbessertes Signal-zu-Rausch-Verhältnis auf. Bei dem vorbekannten Verfahren gemäß dem Artikel von Gleich und Weizenecker wird ein Messsignal sequentiell nur im feldfreien Punkt des angelegten magnetischen Selektionsfeldes erzeugt. Für eine höhere räumliche Auflösung nimmt aufgrund der verstärkten Gradienten das Volumen dieser signalgenerierenden Region ab. Bei einer zweidimensionalen Darstellung nimmt das Signal-zu-Rausch-Verhältnis mit zunehmender Auflösung proportional zu $1/N$, bei einer dreidimensionalen Darstellung proportional zu $1/N^3$ ab, wobei N die Anzahl der Bildpunkte in einer Dimension ist.

Während das herkömmliche Magnetic-Particle-Imaging in jedem einzelnen Messschritt eine Einzelvoxelanregung (Volumen im feldfreien Punkt) untersucht, wird mit dem hier beschriebenen neuen Verfahren mit jeder einzelnen Messung ein Signal aus dem gesamten Probenvolumen aufgenommen. Damit nimmt aber das Signal-zu-Rausch-Verhältnis mit der Anzahl der Messschritte zu. Der damit gegenüber dem bekannten Magnetic-Particle-Imaging erzielte Vorteil ist besonders dramatisch bei einer dreidimensionalen Bildgebung. Wird z.B. eine dreidimensionale Bildmatrix mit 64x64x64 Bildpunkten untersucht, so ergibt sich hieraus ein 512-fach vergrößertes Signal-zu-Rausch-Verhältnis des neuen Verfahrens gegenüber der bislang bekannten Technologie. Wollte man ein 512-fach höheres Signal-zu-Rausch-Verhältnis durch Mittelung erzielen, müsste man mehr als 260.000-fach mitteln. Die wiederum gegenüber dem herkömmlichen Magnetic-Particle-Imaging niedrigere Signalbandbreite erlaubt zudem die Konzeption entsprechend angepasster Empfängerschwingkreise.

Das hier vorgeschlagene neuartige Verfahren eines Magnetic-Particle-Imaging bietet den weiteren Vorteil, dass aus dem Kernspinresonanz-Verfahren bekannte Bildgebungsmethoden adäquat durchgeführt werden können. So können beispielsweise die rotierenden Magnetisierungen durch ein stärkeres rotierendes Magnetfeld oder durch Herabsetzung dessen Frequenz zu einer synchronen Drehung und damit in Phase zueinander gebracht werden, was gewissermaßen dem 90° - Spinflip der Kernspinresonanz entspricht, der in jeder grundlegenden Messsequenz der Kernspintomographie zur Erzeugung eines messbaren Magnetisierungsanteils benötigt wird. Das Maß der Abnahme der superpositionierten Quermagnetisierung ist dann ein Maß für die so genannte Querrelaxation, die das Auseinanderlaufen der einzelnen Magnetisierungen aufgrund der bereits beschriebenen Umgebungsparameter, Feldinhomogenitäten oder Partikelvariationen beschreibt. Ähnlich dem aus dem Kernspinresonanz-Verfahren bekannten Spin-Echo können die einzelnen Magnetisierungen wieder in Phase gebracht werden, indem die Drehrichtung des äußeren magnetischen Feldes umgekehrt wird. Dies entspricht gewissermaßen einem 180°-Spinflip, wie er zum Erzeugen des Spin-Echos in der Kernspinresonanz eingebracht wird.

Als magnetische Kleinstpartikel werden ferromagnetische oder super-paramagnetische Partikel eingesetzt, die im Verhältnis zu ihrem Volumen ein möglichst großes magnetisches Moment aufweisen. Für einen Einsatz in der Biologie und der Medizin kommen insbesondere magnetische Nanopartikel mit Teilchendurchmessern zwischen 20 und 200 nm in Frage. Je größer das Verhältnis zwischen magnetischem Moment und Volumen ist, desto höher ist im Falle der asynchronen Rotation die Rotationsdrift gegenüber dem äußeren magnetischen Feld.

Die Rotationsdiffusion und die Streuung der Partikeleigenschaften führen wie vorbeschrieben zu einer Dephasierung der Partikel, so dass sich die Magnetisierung im Mittel aufhebt. Das vorliegende Verfahren ist damit letztlich durch die Rotationsdiffusion limitiert, da diese die Korrelation der Winkelorientierung der Kleinstpartikel untereinander begrenzt. Um die aus der Kernspintomographie bekannten Konzepte der Frequenz- und Phasenkodierung sinnvoll umsetzen zu können, sollte bevorzugt die Bedingung $mB > \sqrt{2D}$ erfüllt sein. Dabei bezeichnet D eine Diffusionskonstante, die bei einer mechanischen Drehung des Kleinstpartikels insbesondere über die Viskosität und im Falle einer Drehung der Magnetisierung im Kristallgitter wiederum empirisch gegeben ist. Im ersten Fall spricht man von der so genannten Brown-Relaxation. Im letzteren Fall von der Néel-Relaxation. Die gegebene Ungleichung ist ein Maß für die erreichbare Trennschärfe der Frequenz- oder Phasenkodierung. Je größer mB ist, desto kleiner wir die resultierende Linienverbreiterung im Signalspektrum.

Bei einer mechanischen Drehung des Kleinstpartikels kann über den Rotationsdrift die Viskosität der umgebenden Flüssigkeit oder im Falle eines medizinischen Einsatzes eine erhöhte Reibung mit Gefäß- oder Zellwänden beobachtet werden. Da Temperaturunterschiede sich in unterschiedlicher Viskosität bemerkbar machen, kann innerhalb eines Mediums über die Viskosität auch die Temperatur gemessen werden. Wenn die Kleinstpartikel als Sonden eingesetzt sind, an deren Oberfläche Moleküle angelagert sind, vergrößert sich der hydrodynamische Teilchendurchmesser des Kleinstpartikels und dementsprechend verringert sich die Rotationsdrift. Aus diesem Grund lassen sich über die Rotationsdriftverschiebung Molekülkonzentrationen lokalisieren und quantifizieren.

Wenn sich hingegen das Kleinstpartikel an Zelloberflächen anlagert, wird die Brown-Relaxation praktisch vollständig unterdrückt, so dass nur noch die Néel-Relaxation vorliegt. Dadurch kann sich je nach Partikelsorte die Relaxationsdauer um mehrere Größenordnungen ändern. Im Falle von Magnetit-Nanopartikeln mit einem mittleren Durchmesser von etwa 25 nm ergibt sich damit eine Änderung der Relaxationsdauer von ca. 5 Mikrosekunden zu ca. 1 Millisekunde.

Die magnetische Kopplung von verclusterten Kleinstpartikeln, beispielsweise durch ein Cross-Linking über funktionale Gruppen oder Agglomeration in Zellvesikeln, verändert ebenfalls den Rotationsdrift und kann darüber detektiert werden.

Vorteilhafterweise wird während der Erfassung der Frequenzanteile der Quermagnetisierung ein magnetisches Gradientenfeld zu einer Frequenzkodierung der Ortsinformation erzeugt. Bei der Messung der Quermagnetisierung wird ein Frequenzspektrum erfasst, in dem jeder Frequenz eine räumliche Koordinate entlang des Gradientenfeldes zugeordnet ist. Die räumliche Auflösung ist dabei durch die erzielbare örtliche Variation des Rotationsdrifts gegeben. Das Produkt aus Einschaltdauer und Gradientenstärke sollte bevorzugt so gewählt sein, dass auf der kleinsten Distanz, die noch aufgelöst werden soll, sich die Orientierung der Magnetisierung ab dieser Distanz um mindestens eine volle Umdrehung am Ende der Messung unterscheidet.

Feldinhomogenitäten, die Streuung der Partikeleigenschaften und örtliche Variationen in Bezug auf die chemische Umgebung, die Viskosität oder ähnliches verursachen eine spektrale Verbreiterung, die die Ortskodierung mittels Frequenzzuordnung limitiert. Wenn die Streuung der Partikeleigenschaften zu hoch ist oder wenn zusätzlich einer oder mehrere der weiteren angesprochenen Parameter wie die Viskosität oder die Temperatur oder Molekülkonzentrationen über die Frequenzen der asynchronen Rotation abgefragt werden sollen, kann die Frequenzkodierung nicht uneingeschränkt zur Ortskodierung herangezogen werden.

Diese Limitierung kann umgangen werden, indem die Frequenzkodierung um Phasenkodierschritte ergänzt oder vollständig durch eine Phasenkodierung ersetzt wird. Vorteilhafterweise wird daher insbesondere vor der Erfassung der Frequenzanteile der Quermagnetisierung ein magnetisches Gradientenfeld für eine vorgegebene Zeitspanne erzeugt, um eine Phasenkodierung im Partikelensemble zu erzeugen.

Wenn das Messsignal nach der Phasenkodierung erfasst wird, hat jeder Ort eine der Position im gewählten Gradienten und dessen Stärke entsprechende Phasenverschiebung erfahren. Insbesondere liefern beispielsweise N Schritte mit jeweiliger Erhöhung der Gradientenstärke um 1/N der maximal möglichen Gradientenstärke die Information, um aus einer ortsabhängigen Phasenverschiebung oder Phasenlage auf die Verteilung der Quermagnetisierung zu N Koordinaten rückrechnen zu können. Dies wird bevorzugt mittels einer schnellen Fouriertransformation (FFT) vorgenommen.

Die Quermagnetisierung kann grundsätzlich auch während der Phasenkodierschritte erfasst werden. Die so gewonnenen Daten lassen sich jedoch nicht einem kartesisch geordneten Fourierraum zuordnen, aus dem sich die gewünschte räumliche Verteilung direkt durch eine Fouriertransformation berechnen ließe. Vielmehr liefern die Daten komplementäre Informationen und lassen sich nur gesondert und somit aufwändig auswerten.

Wird die Bildkodierung vollständig durch Phasenkodierung vorgenommen, so werden für 3 Dimensionen mit jeweils N Koordinaten NxNxN Messschritte benötigt. In diesem Fall liefert die Frequenzverteilung, die insbesondere im Anschluss nach jedem Schritt ohne Gradientenfeld erfasst wird, vollkommen redundante Information, die in erster Linie die Verteilung oder Streuung der Rotationseigenschaften der Kleinstpartikel wiederspiegelt. Diese Verteilung erhält man in diesem Fall für jeden der NxNxN Raumpunkte separat. Da diese Verteilung unabhängig vermessen werden kann, lässt sich aus der Verschiebung dieser Frequenzverteilung auf eine Ortsverteilung der Umgebungsparameter wie z.B. der Temperatur schließen.

Nach der Phasenkodierung kann selbstverständlich wieder ein magnetisches Gradientenfeld zu einer Frequenzkodierung angelegt werden, um beispielsweise hierdurch eine Ortskodierung in der anderen Raumrichtung zu erhalten. Dies bietet sich an, wenn nur die Dichteverteilung der Kleinstpartikel gefragt ist, und wenn wie erwähnt, die Variation der Partikeleigenschaften hinreichend klein ist.

Statt die Frequenzkodierung vollständig durch Phasenkodierung zu ersetzen, kann die Kodierung demnach auch nur zum Teil durch Phasenkodierung und zum Teil durch Frequenzkodierung erfolgen. Die Anzahl der zusätzlich benötigten Unterteilung der Frequenzkodierung in Phasenkodierschritte ergibt sich beispielsweise aus der jeweiligen Breite der Partikelstreuung bzw. der daraus resultierenden Spektralverbreiterung.

Die Abbildung einer räumlichen Verteilung des Partikelensembles gegenüber der Abbildung einer räumlichen Verteilung der Umgebungsparameter unterscheidet sich darin, dass die Frequenzkodierung einmal zur Ortsauflösung herangezogen wird, und einmal die vorhandene Frequenzverteilung zur Erfassung der durch die Umgebung aufgeprägte Variation genutzt wird. Im ersten Fall wird während der Messung der Gradient zur Ortskodierung der Frequenzen eingeschaltet. Im zweiten Fall findet die Signalerfassung ohne eingeschalteten Gradienten statt.

In einer zweckmäßigen Ausgestaltung wird nach Abschalten des magnetischen Gradientenfeldes ein zweites gleiches magnetisches Gradientenfeld umgekehrten Vorzeichens mit derselben Zeitspanne erzeugt, wobei danach in einer Ebene senkrecht zum Verlauf des Gradienten die ortsaufgelöste Verteilung der Quermagnetisierung ermittelt und ausgegeben wird. Dieses Verfahren ermöglicht die Durchführung einer räumlichen Vermessung von Flussprofilen oder Gefäßstrukturen.

Dabei wird für eine vorbestimmte Zeitdauer zunächst ein magnetischer Gradient in einer vorgegebenen Raumrichtung, in der der Fluss gemessen werden soll, angelegt. Anschließend wird für die gleiche Zeitdauer derselbe Gradient mit derselben Gradientenstärke, jedoch mit umgekehrtem Vorzeichen angelegt. In einer Ebene senkrecht zum Gradienten erfolgt die räumliche Bildkodierung wie bereits beschrieben, beispielsweise mittels geeigneter Frequenzkodierung und/oder Phasenkodierung. Die Frequenzverteilung der Quermagnetisierung wird erfasst. Die ersten beiden Schritte haben den Effekt, dass unbewegte Kleinstpartikel keine Phasenänderung erfahren, da der zweite Schritt für ruhende Partikel den ersten Schritt quasi vollständig rückgängig macht. Sich in Richtung des Gradienten bewegende Partikel erfahren jedoch eine Phasenänderung, die proportional zur zurückgelegten Wegstrecke ist. Die Phasenänderung ist damit ein Maß für die Flussgeschwindigkeit.

Um eine Flussmessung auch mit Partikeln mit großer Streuung der Rotationseigenschaften durchführen zu können, ist eine Kombination mit einem 180°-Spinflip vorteilhaft. Nach einem noch zu erläuterndem Refokusierungspuls zur "Gleichphasierung" des Partikelensembles werden die ersten beiden Schritte einmal vor und einmal nach einer Umkehr der Rotationsrichtung des magnetischen Rotationsfeldes durchgeführt. Danach ergibt sich ein Echosignal, dass bei ruhendem Partikel bei genau der doppelten Zeit zwischen Refokusierungspuls und Drehrichtungsumkehr auftritt. Die inzwischen aufgrund der Partikeleigenschaften außer Phase geratenen Kleinstpartikel rotieren nach Drehrichtungsumkehr wieder in Phase, woraus ein in der Intensität wieder angewachsenes Echosignal der superpositionierten Quermagnetisierung resultiert. Wenn sich die Kleinstpartikel oder das Partikelensemble in Richtung des Gradienten bewegt, dann verschiebt sich dieses Echosignal proportional zur Geschwindigkeit, wobei jeglicher Einfluss aufgrund von Partikelstreuung neutralisiert ist.

In einer bevorzugten Weiterbildung des angegebenen Verfahrens wird ein magnetisches Gradientenfeld durch räumliche Variation der Feldstärke des rotierenden Magnetfeldes erzeugt. Das rotierende Magnetfeld kann beispielsweise über in Umfangsrichtung verteilte Spulenpaare erzeugt werden, die entsprechend angesteuert werden. In der einfachsten Ausführung kann das rotierende Magnetfeld über ein Paar von orthogonal zueinander ausgerichteten Helmholtzspulen erzeugt werden. Dazu werden die Helmholtzspulenpaare entsprechend sinusförmig angesteuert. Über eine veränderte Ansteuerung kann über diese Spulen auch ein zusätzliches Gradientenfeld zur Ortskodierung erzeugt werden. Im einfachsten Falle ist demnach keine separate Gradientenspule zur Erzeugung des magnetischen Gradientenfeldes erforderlich.

In einer zusätzlichen oder alternativen Ausgestaltung wird das Gradientenfeld nicht durch eine Variation der Feldstärke des rotierenden magnetischen Feldes, sondern durch Überlagerung eines homogenen Rotationsfeldes mit einem statischen, räumlich variierenden Offsetfeld erzeugt. Dieses wird beispielsweise mit separaten Gradientenspulen generiert.

Über das magnetische Gradientenfeld wird der mittleren asynchronen Rotation der einzelnen Magnetisierungen und damit insbesondere der messbaren superpositionierten Quermagnetisierung eine Ortsabhängigkeit aufgeprägt. Diese lässt sich - wie bereits beschrieben - unmittelbar zu einer eindimensionalen räumlichen Abbildung der entsprechenden Verteilung der Kleinstpartikel nutzen. Um Umgebungsparameter, chemische Veränderungen, etc. lokalisieren und entsprechend abbilden zu können, wird in einer besonderen Ausführungsform das beschriebene Verfahren wiederholt, wobei die Frequenzanteile der Quermagnetisierung mehrmals für verschiedene Gradientenfelder erfasst werden. Hierdurch können die asynchronen Rotationsfrequenzen lokalisiert, entsprechend analysiert und zur Bildgebung verwendet werden.

In einer weiter bevorzugten Ausgestaltung wird in einem ersten Schritt für eine Zeitspanne ein erstes Gradientenfeld entlang einer ersten Richtung erzeugt, in einem zweiten Schritt ein zweites Gradientenfeld entlang einer zweiten Richtung erzeugt, in einem dritten Schritt die Frequenzverteilung der Quermagnetisierung erfasst, in einem vierten Schritt die vorgenannten Schritte 1 bis 3 mit einer Anzahl abgeänderter erster Gradientenfelder wiederholt und aus dem gewonnenen Satz an Messsignalen eine zweidimensionale Verteilung der Quermagnetisierung ermittelt und ausgegeben.

Bei dieser Methode zur zweidimensionalen Darstellung wird der Frequenzraum mit einem Raster kartesischer Koordinaten abgetastet. Dabei wird zu Beginn der Messung über das erste Gradientenfeld entlang der ersten Richtung der Magnetisierung der Kleinstpartikel ein Phasengang aufgeprägt, indem das erste Gradientenfeld für eine vorgegebene Zeit über das Messvolumen gelegt wird. Die solcherart vorgeprägte Quermagnetisierung wird dann über ein zweites Gradientenfeld auf die zweite Richtung projiziert. Die Wiederholung dieser Messsequenz mit unterschiedlicher Vorphasierung durch Variation des ersten Gradientenfelds liefert dann einen Satz von Messsignalen, der einem Hologramm entspricht. Durch eine zweidimensionale Fourier-Transformation kann dann unmittelbar die gewünschte räumliche Verteilung der Magnetisierung erhalten werden. Dies ermöglicht eine direkte Bildrekonstruktion mit Hilfe einer zweidimensionalen "Fast-Fourier-Transformation (FFT)" innerhalb kürzester Zeit.

Wird das zweite Gradientenfeld nicht zu einer Frequenzkodierung während der Messung eingeschaltet, sondern zu einer vorhergehenden Phasenkodierung verwendet, so kann wie bereits beschrieben, eine räumliche Verteilung der Umgebungsparameter erhalten werden. Dabei können das erste und das zweite Gradientenfeld auch zeitgleich angelegt werden, da sich der Effekt der Gradienten auf die Phase annähernd linear superpositioniert.

Wird in diesem Verfahren das erste und das zweite Gradientenfeld zur Phasenkodierung vor der Messung verwendet und ein drittes Gradientenfeld zu einer Frequenzkodierung während der Messung eingesetzt, so kodieren die ersten beiden ersten Gradientenfelder zwei Raumrichtungen über die Phase und das dritte Gradientenfeld die dritte Raumrichtung über die Frequenz. Dies entspricht dann einer dreidimensionalen Bildgebung einer räumlichen Verteilung des Partikelensembles.

Für eine dreidimensionale Darstellung wird dieses Verfahren insofern weitergebildet, wobei nach dem zweiten Schritt ein drittes Gradientenfeld entlang einer dritten Richtung erzeugt wird, wobei die Schritte 1 bis 4 mit einer Anzahl abgeänderter zweiter Gradientenfelder wiederholt werden, und wobei aus dem Satz an gewonnenen Messsignalen eine dreidimensionale Verteilung der Quermagnetisierung ermittelt und ausgegeben wird. Bevorzugt stehen in beiden Verfahren die jeweiligen Raumrichtungen x, y und z senkrecht aufeinander.

Werden im letztgenannten Verfahren alle drei Gradientenfelder vor der Datenerfassung zur Phasenkodierung eingesetzt, so werden alle drei Raumrichtungen über die Phase erfasst. Insgesamt sind somit für eine 3D-Bildmatrix aus NxNxN Bildpunkten genauso viele Messschritte notwendig. Dafür enthält aber die Frequenzverteilung nun die Information über die Variation der Rotationseigenschaften für jeden Voxel separat, so dass erstens die erzielbare Auflösung nicht mehr durch die Partikeldispersion limitiert ist, und zweitens die Umgebungsparameter wie Temperatur, Molekülkonzentrationen usw. räumlich aufgelöst werden können.

Davon unbenommen verbleibt für das vorliegende Verfahren auch die Möglichkeit, zur Bildrekonstruktion den Frequenzraum mit einem Raster in Polarkoordinaten abzutasten.

In einer bevorzugten Ausgestaltung wird aus der ermittelten Verteilung der Quermagnetisierung eine ortsaufgelöste Dichteverteilung der Kleinstpartikel ermittelt und ausgegeben. Bei einer Ortskodierung der mittleren asynchronen Rotationsfrequenzen durch ein auferlegtes magnetisches Gradientenfeld folgt die ortsaufgelöste Dichteverteilung der Kleinstpartikel unmittelbar aus den Amplituden der entsprechenden Frequenzanteile der Quermagnetisierung.

Um eine hohe Signalausbeute zu erhalten, und um insbesondere die vorbeschriebene Phasenkodierung zur mehrdimensionalen Bildgebung zu erzielen, werden zweckmäßigerweise vor Messung mittels eines kurzzeitigen magnetischen Refokussierungsfelds die Magnetisierungen der Kleinstpartikel in eine zum rotierenden Magnetfeld synchrone Rotation versetzt. Unmittelbar nach Abschalten dieses Refokussierungsfelds rotieren alle Magnetisierungen dann in Phase. Das Messsignal der superpositionierten Quermagnetisierung wird maximal. Durch Anlegen eines kurzzeitigen Gradientenfeldes kann dann, ausgehend von dieser Situation, die Phasenkodierung vorgenommen werden. Ebenso kann ausgehend von dieser Situation in einem zur asynchronen Drehung angelegten äußeren Magnetfeld das Auseinanderlaufen der einzelnen Magnetisierungen aufgrund der unterschiedlichen chemischen Einbettung oder Umgebung beobachtet werden. Mit zunehmender Zeitspanne dephasieren die einzelnen asynchron rotierenden Magnetisierungen der Kleinstpartikel.

Das Refokussierungsfeld kann entweder durch eine kurzzeitige Erhöhung der magnetischen Feldstärke und/oder durch eine kurzzeitige Erniedrigung der Rotationsfrequenz des rotierenden Magnetfelds erzeugt werden. Alternativ kann ein kurzzeitig hinzugeschaltetes magnetisches Offsetfeld erzeugt werden.

In einer weiter vorteilhaften Ausgestaltung wird das Refokussierungsfeld derart erzeugt, dass selektiv nur die Magnetisierungen von Kleinstpartikeln gleicher Rotationseigenschaften in eine synchrone Rotation versetzt werden. Dabei wird die Tatsache ausgenutzt, dass die beschriebene kritische Frequenz $\Omega_c$, die den Übergang zwischen synchroner und asynchroner Rotation beschreibt, auch vom Volumen bzw. der Form der Kleinstpartikel abhängig ist. Diese Abhängigkeit führt zum einen dazu, dass das Messsignal der erfassten superpositionierten Quermagnetisierung sich bei zunehmender Bandbreite der Kleinstpartikelvariation verschlechtert. Wird auf der anderen Seite der Refokussierungspuls so erzeugt, dass nur Kleinstpartikel gleicher Rotationseigenschaften in eine synchrone Rotation versetzt werden, so tragen nur diese zur superpositionierten Quermagnetisierung bei. Die Magnetisierungen der anderen Kleinstpartikel sind hingegen stochastisch verteilt. Insgesamt wird auf diese Weise eine Verbesserung des erfassten Messsignals erhalten, ohne dass die Bandbreite der Größen der eingesetzten Kleinstpartikel verringert werden muss, was gegebenenfalls mit hohen Kosten verbunden ist.

In einer besonderes vorteilhaften Ausgestaltungsvariante wird zu einer Phasenkodierung ein rotierendes magnetisches Gradientenfeld mit seinem Gradientenverlauf räumlich über das Meßvolumen geschoben, wobei die Feldstärke im Gradientenverlauf über einen Wert, der zur Anregung einer asynchronen Rotation der Kleinstpartikel führt, bis wenigstens zu einem Wert, der zur Anregung einer synchronen Rotation der Kleinstpartikel führt, ansteigt. Dabei wird berücksichtigt, dass die Feldabhängigkeit der asynchronen Rotationsdrift nicht linear verläuft, und dass magnetische Kleinstpartikel typischerweise in ihren Partikeleigenschaften streuen.

Wird, wie vorstehend bereits beschrieben, eine Phasenkodierung durch Anlegen eines magnetischen Gradientenfelds für eine bestimmte Zeitdauer vorgenommen, so führen die nicht lineare Abhängigkeit der asynchronen Rotationsdrift von der Feldstärke sowie die Streuung der Partikeleigenschaften zu einer unerwünschten Verschlechterung der Bildauflösung. Die eingeprägte örtliche Phasenlage ist verschmiert. Sich in ihren Partikeleigenschaften unterscheidende Kleinstpartikel erfahren am selben Ort eine unterschiedliche Phaseneinprägung. Insbesondere unterscheidet sich auch der Phasenverlauf über das Messvolumen.

Wird stattdessen zur Phasenkodierung der Gradientenverlauf eines rotierenden magnetischen Gradientenfelds über das Messvolumen geschoben, und steigt die Feldstärke im Gradientenverlauf bis wenigstens zu einem Wert, bei oder ab dem die Kleinstpartikel zu einer synchronen Rotation angeregt werden, so wird allen Kleinstpartikeln über das Messvolumen derselbe Phasenverlauf eingeprägt. Unterschiedliche Partikelsorten weisen zueinander lediglich einen global gleichbleibenden Phasenversatz auf. Diese Phasendifferenz beeinträchtigt jedoch die Linearität der Abbildung nicht. Stattdessen enthält die Phasendifferenz zusätzliche Information über die Partikelverteilung oder andere lokale Unterschiede, was separat ausgewertet werden kann.

Dazu werden beispielhaft zwei Partikelsorten (oder zwei identische Partikel in unterschiedlicher Umgebung) betrachtet, die bei gegebener Rotationsrate des Gradientenfeldes eine unterschiedliche kritische Feldstärke aufweisen. Bei Unterschreiten der kritischen Feldstärke reißt die synchrone Rotation ab. Im Bereich des Gradientenfeldes mit hoher Feldstärke rotieren beide Partikelsorten synchron zur Rotation des äußeren Feldes. Wird der Gradientenverlauf nun räumlich über das Messvolumen geschoben, so wird zunächst für die Partikelsorte mit höherer kritischer Feldstärke diese unterschritten, und erst zeitlich versetzt dazu die der Partikelsorte mit geringerer kritischer Feldstärke. Mit anderen Worten erfahren beide Partikelsorten eine zeitlich versetzte Abschaltung des Rotationsfeldes. Der Zeitversatz ist dabei von der Form des Gradientenverlaufs und von der Verschiebungsrate abhängig. Über das Messvolumen bzw. in Kodierrichtung erfahren beide Partikelsorten die Aufprägung desselben Phasenverlaufs. Die Phasen beider Partikel unterscheiden sich global in einer festen Phasendifferenz.

Die Verschiebung des beschriebenen Gradientenverlaufs eines rotierenden Magnetfeldes ermöglicht es somit, unabhängig von Partikeleigenschaften und der chemischen Umgebung, eine Ortskodierung durch Einprägung eines Phasenverlaufs vorzunehmen. Die Einprägung einer sogenannten räumlichen Harmonischen mit einer räumlichen Wellenlänge, entlang derer die Phasenlage sich um 180° verändert, führt bei Messung der makroskopischen Quermagnetisierung zur Bestimmung des entsprechenden Fourierkoeffizienten. Durch entsprechende Wiederholung der Phasenkodierung mit verschiedener Verschiebungsrate können die Fourierkoeffizienten der anderen Harmonischen ermittelt werden, so dass die vollständige Fourierkodierung erhalten wird. Beim N-ten Phasenkodierschritt entsprechend N Bildpunkten in Kodierrichtung dreht sich die Richtung des Magnetisierungsvektors im Messvolumen N/2-mal um die eigene Achse. Zu einer dreidimensionalen Bildgebung müssen entsprechend NxNxN Schritte durchgeführt werden.

Das beschriebene Verfahren verknüpft die Refokussierung mit der Aufprägung einer Phase. Beim Überfahren des Messvolumens mit dem Gradientenverlauf des rotierenden magnetischen Gradientenfeldes werden Feldstärken entsprechend einer Refokussierung wirksam, die die Kleinstpartikel zu einer synchronen Rotation anregen. Beim weiteren Verschieben unterschreitet die Feldstärke den kritischen Wert, so dass danach das Regime der asynchronen Rotation erreicht wird.

Bevorzugt steigt die Feldstärke im Gradientenverlauf von im Wesentlichen Null an. Praktisch gibt es aber eine untere Grenze der Feldstärke, ab der sich nur noch minimale Kodierfehler ergeben.

Die Verschiebung des Gradientenverlaufs erfolgt zweckmäßigerweise durch eine individuelle Ansteuerung einzelner Spulen in einem Spulenarray. Insbesondere wird hierzu entlang der Kodierrichtung ein zeitlich versetztes Ein- und Abschalten der individuellen Spulen vorgenommen.

Bei Partikeln mit Neelrelaxation ist effektiv die Größe das entscheidende Selektionskriterium. Entscheidend ist generell das Verhältnis aus magnetischem Moment des Partikels und seinem Reibungsterm. Bei massiven Partikeln mit im Gitter fixierter Magnetisierung ist der Reibungsterm unabhängig von der Größe, sondern hängt nur vom Formfaktor ab. Durch die geeignete Wahl der Feldstärke und/oder der Rotationsfrequenz des Refokusierungsfeldes werden demnach Partikel gleicher Rotationseigenschaften, d.h. Partikel mit gleicher Rotationsdrift bei gleicher Feldstärke und gleicher Rotationsfrequenz, selektiert.

Eine solche selektive Refokusierung kann beispielsweise durch ein rotierendes Magnetfeld erzielt werden, dessen Drehachse auf einer Trajektorie umläuft. In diesem Fall gibt es nur für ganz bestimmte Partikel mit einem ganzzahligen Teilerverhältnis ihrer Rotationsfrequenz eine Refokusierung. Denn nur solche Partikel gelangen nach Abschluss der Anwendung des 3D-Rotationsfeldes wieder in ihre Ausgangsposition zurück, die eine 1/n-fache Rotationsfrequenz gegenüber der Rotation des Refokusierungsfeldes aufweisen (n: ganzzahlig). Auf diese Weise können hochselektive Refokusierungspulse erreicht werden. Die Signalselektion erfolgt durch einen geeigneten Tief- oder Bandpaßfilter.

Die Bandbreite der erfassten Quermagnetisierung kann bevorzugt durch eine entsprechende Einstellung der Feldstärke und/oder der Rotationsfrequenz des rotierenden Magnetfelds und/oder einer Einstellung des Gradientenfelds (Steigung, Feldstärke) verringert werden. Die Signalerfassung wird hierdurch verbessert.

In einer zweckmäßigen Ausgestaltung wird das oder jedes magnetische Gradientenfeld derart erzeugt, dass sich eine lineare Ortsabhängigkeit der mittleren Rotationsfrequenz der Magnetisierungen ergibt. Mit anderen Worten wird das magnetische Gradientenfeld gewissermaßen invers zur gegebenen Abhängigkeit der mittleren Rotationsfrequenz von der Feldstärke eingestellt, so dass sich im Umkehrschluss eine lineare Abhängigkeit der mittleren Rotationsfrequenz vom Ort ergibt. Dies erlaubt eine raschere Bildrekonstruktion.

Die superpositionierte Quermagnetisierung kann grundsätzlich über geeignete physikalische Messmethoden abgegriffen werden. Insbesondere eignet sich ein induktiver Abgriff über eine geeignet ausgebildete und ausgerichtete Messspule.

Da die zu dem neuen Verfahren des Magnetic-Particle-Imaging benötigten Feldstärken im Milli-Tesla-Bereich (mT) liegen, eröffnet sich eine zur physiologischen Bildzuordnung einzigartige Möglichkeit, nämlich das hier angegebene Verfahren mit einem Low-Field-Kernspinresonanz-Verfahren zu kombinieren. Das zur Bildkodierung verwendete Gradientenfeld, welches mit Luftspulen erzeugt werden kann, lässt sich nämlich beliebig ausschalten. Das für die bisherige Methode der Bildgebung mittels magnetischer Kleinstpartikel verwendete Verfahren benötigt hingegen ein enorm starkes magnetisches Gradientenfeld, welches sich aufgrund der notwendigen Neodym-Dauermagnete nicht ausschalten lässt. Eine Kombination mit einem Kernspinresonanz-Verfahren scheidet daher aus.

Da ein Verfahren zur Bildgebung mittels magnetischer Kleinstpartikel ausnahmslos Kontrastmittel abbildet, wird aber insbesondere im medizinischen Bereich eine physiologische Bildzuordnung unerlässlich. Vorliegend kann diese physiologische Bildzuordnung nun insbesondere durch die parallele Bereitstellung eines Low-Field-Kernspinresonanz-Verfahrens erzielt werden. Das hier angegebene Verfahren zur Bildgebung aus der asynchronen Drehung von magnetischen Kleinstpartikeln und das Low-Field-Kernspinresonanz-Verfahren können dazu problemlos in einem einzigen Gerät verwirklicht werden. Denn auch das Low-Field-Kernspinresonanz-Verfahren arbeitet bei Feldstärken, die sich mit üblichen Elektromagneten, insbesondere mit Luftspulen, erzeugen lassen. Zu einer medizinischen Untersuchung kann dann die Bildgebung des Low-Field-Kernspinresonanz-Verfahrens über die Abbildung von Organen, Geweben, etc. zur physiologischen Zuordnung der aufgenommenen Verteilung des Kontrastmittels mit magnetischen Kleinstpartikeln verwendet werden.

Hinsichtlich der Vorrichtung wird die gestellte Aufgabe erfindungsgemäß gelöst durch die Merkmale des Anspruchs 14.

Die Ausgabevorrichtung kann dabei die gewonnenen Messdaten bzw. die gewonnene ortsaufgelöste Verteilung beispielsweise in Form von digitalen Daten, als Speicherinformation auf Datenträgern, über Netzwerke oder dergleichen zur Verfügung stellen, oder unmittelbar ein Informationsträger sein, auf den die gewonnene ortsaufgelöste Verteilung dargestellt wird.

In einer zweckmäßigen Ausgestaltung umfasst die Messeinrichtung eine parallel zur Längsachse ausgerichtete Induktionsspule. Diese kann insbesondere als eine Solenoid-Spule mit mehreren parallelen Windungen ausgestaltet sein. Über eine solche Induktionsspule wird dann unmittelbar die superpositionierte Quermagnetisierung als Messsignal erfasst. Bevorzugt ist die Messeinrichtung durch ein Array von Detektorspulen gebildet, wobei jedes Spulensegment des Arrays seinen eigenen Empfangskanal besitzt. Da sich jedes Spulensegment an einer anderen räumlichen Position befindet, ergibt sich aufgrund des räumlich begrenzten Empfangsprofils eines einzelnen Spulensegments bereits eine grobe räumliche Kodierung. Dadurch lässt sich die Bildkodierung mit entsprechend weniger Phasenkodierschritten durchführen, wodurch sich die Bildakquisition beschleunigen lässt. Für jedes Spulensegment ergibt sich gewissermaßen ein unvollständiger Datensatz (wegen der fehlenden Phasenkodierung), aus dem sich grundsätzlich nun unter Verwendung der Zusatzinformation des Array ein einziger, vollständiger Datensatz rekonstruieren lässt. Zur Bilderzeugung müssen die Daten fouriertransformiert werden. Die Rekonstruktion kann insofern vor der Transformation unter Zuhilfenahme der Zusatzinformation des Array erfolgen oder nach der Transformation durch eine entsprechende Entfaltung vorgenommen werden.

Zur Unterdrückung von durch das äußere Magnetfeld erzeugten Induktionsspannungen oder -strömen im Messsignal ist das Eingangssignal des Magnetfeldgenerators bevorzugt mit dem Spulenausgangssignal gegenphasig gekoppelt. Diese Kopplung kann beispielsweise induktiv mittels eines Transformators oder dergleichen erfolgen.

Bevorzugt wird auch eine aktive Gegenkopplung zur Verbesserung des Messverfahrens eingesetzt. Dabei werden die Störanteile in der Empfangskette in einer Kalibriermessung erfasst. Diese Störanteile werden dann in der eigentlichen Messung mit entgegengesetztem Vorzeichen direkt in die Empfangskette eingekoppelt. Dazu ist vorteilhafterweise ein Transformator oder allgemein ein HF-Übertrager vorgesehen. Das Einkopplungssignal wird insbesondere mittels eine Digital-Analog-Konverter generiert.

Zur Entfernung der nicht zum Messsignal beitragenden höheren Frequenzen der superpositionierten Quermagnetisierung, die aus Rotationsflips gegenüber dem äußeren rotierenden Magnetfeld herrühren, ist zweckmäßigerweise ein Tief- oder Bandpassfilter vorgesehen. Durch einen Tiefpassfilter werden Frequenzen unterhalb der Rotationsfrequenz des äußeren rotierenden Magnetfelds selektiert. Ein Bandpassfilter ermöglicht es auch, niederfrequente Störanteile auszublenden. Solche niederfrequente Störanteile können beispielsweise durch periodische vorgenommene Refokusierungen vor jeder Messsequenz auftreten.

In einer vorteilhaften Ausgestaltung umfasst der Magnetfeldgenerator normal leitende Spulenpaare, die zur Erzeugung eines rotierenden Magnetfeldes mit einer Feldstärke zwischen 0,1 mT und 100 mT und einer Rotationsfrequenz zwischen 1 kHz und 1 MHz ausgelegt sind. Derartige Spulen sind relativ günstig zu beziehen. Die gewünschten Magnetfelder lassen sich mit Luftspulen oder mit wassergekühlten Spulen erzeugen.

In einfacher Ausführung sind die Spulenpaare als orthogonal zueinander ausgerichtete Helmholtzspulenpaare ausgebildet. Dadurch lässt sich in einfacher Art und Weise ein rotierendes äußeres Magnetfeld mit hoher Homogenität erzeugen.

Zur Erzeugung des Gradientenfeldes kann der Magnetfeldgenerator entsprechend angesteuert werden. In einer vorteilhaften Weiterbildung ist wenigstens eine Gradientenspule zur Erzeugung eines magnetischen Gradientenfeldes in Querrichtung zum rotierenden Magnetfeld mit einer Feldstärke zwischen 10 mT und 100 mT vorgesehen. Zur Erzeugung eines linearen Gradienten ist insbesondere eine Golay-Biplanarspule bevorzugt. Im Gegensatz zur Kernspinresonanz entfällt der Hauptmagnet, der dort eine Zylindergeometrie erzwingt. Für die hier beschriebene Messvorrichtung ist daher die biplanare Ausführung die vorteilhafteste Ausführung einer Golay-Spule.

In einer besonders vorteilhaften Ausgestaltung ist die Vorrichtung zur Bildgebung mittels magnetischer Kleinstpartikel kombiniert mit einer hierzu relativ positionierten Vorrichtung zur Durchführung eines Low-Field-Kernspinresonanz-Verfahrens. Auf diese Weise kann eine physiologische Bildzuordnung vorgenommen werden.

Weitere Vorteile lassen sich sinngemäß aus dem beschriebenen Verfahren auf die hier angegebene Vorrichtung übertragen.

Ausführungsbeispiele der Erfindung sind anhand einer Zeichnung näher erläutert. Dabei zeigen:

Fig. 1     den Frequenzgang der mittleren Rotationsfrequenz der Magnetisierung eines Kleinstpartikels gegenüber der Rotationsfrequenz eines äußeren Magnetfeldes,

Fig. 2     den Verlauf der superpositionierten Quermagnetisierung nach einem Refokusierungspuls und

Fig. 3     schematisch eine geeignete Vorrichtung zur Bildgebung mittels magnetischer Kleinstpartikel.

In Fig. 1 ist der Verlauf der mittleren Rotationsfrequenz der Magnetisierung eines magnetischen Kleinstpartikels gegenüber der Rotationsfrequenz eines äußeren magnetischen Feldes dargestellt. Dabei entspricht der Verlauf der Kurve a gegenüber dem Verlauf der Kurve b einer niedrigeren Feldstärke.

Man erkennt, dass die mittlere Rotationsfrequenz der Magnetisierung eines magnetischen Kleinstpartikels bis zu einer kritischen Frequenz $\Omega_c$ synchron mit der äußeren Rotationsfrequenz dreht. Oberhalb der kritischen Frequenz $\Omega_c$ verringert sich die mittlere Rotationsfrequenz gegenüber der äußeren Rotationsfrequenz. Die Magnetisierung oder der Kleinstpartikel selbst rotiert dann mit einer mittleren Rotationsfrequenz asynchron gegenüber dem äußeren Feld. Die Tatsache, dass die kritische Frequenz $Q_c$ und damit auch die mittlere Rotationsdrift gegenüber dem äußeren magnetischen Feld eine Feldabhängigkeit zeigen, lässt sich zur Bildgebung verwenden.

Die Kurven a und b in Fig. 1 können ebenso gut zur Veranschaulichung der Abhängigkeit der mittleren Rotationsdrift gegenüber einer veränderten chemischen Einbettung oder Umgebung herangezogen werden. Beispielsweise würde sich der Kurvenverlauf a bei einer mechanischen Drehung des Kleinstpartikels in einem Medium höherer Viskosität gegenüber dem Kurvenverlauf b ergeben. Bei gleicher Feldstärke kann der Kleinstpartikel im höher viskosen Medium bereits bei einer niedrigeren äußeren Rotationsfrequenz dem Feld nicht mehr folgen.

In Fig. 2 ist der zeitliche Verlauf der gemessenen superpositionierten Quermagnetisierung $M_Q$ nach einem Refokussierungspuls mittels eines kurzzeitig angelegten äußeren Magnetfeldes gezeigt. Nach Abschalten des Refokussierungsfeldes rotieren die Einzelmagnetisierungen der Kleinstpartikel in einem entsprechen angelegten rotierenden, äußeren Magnetfeld in Phase. Das superpositionierte Signal der Quermagnetisierung $M_Q$ weist einen maximalen Wert auf. Entsprechend der Rotationsfrequenz des äußeren Magnetfeldes ergibt sich das dargestellte Summensignal. Infolge von Variationen der Partikelgröße und/oder Partikelform, infolge von Feldinhomogenitäten und infolge von unterschiedlichen chemischen Umgebungen dephasieren die Einzelmagnetisierungen zusehends. Die Amplitude des Messsignals fällt mit einer Relaxationszeit $\tau$ ab. Durch Drehrichtungsumkehr des äußeren Magnetfeldes könnte ein Echo im Messsignal ähnlich dem aus dem Kernspinresonanz-Verfahren bekannten Spin-Echo erzeugt werden.

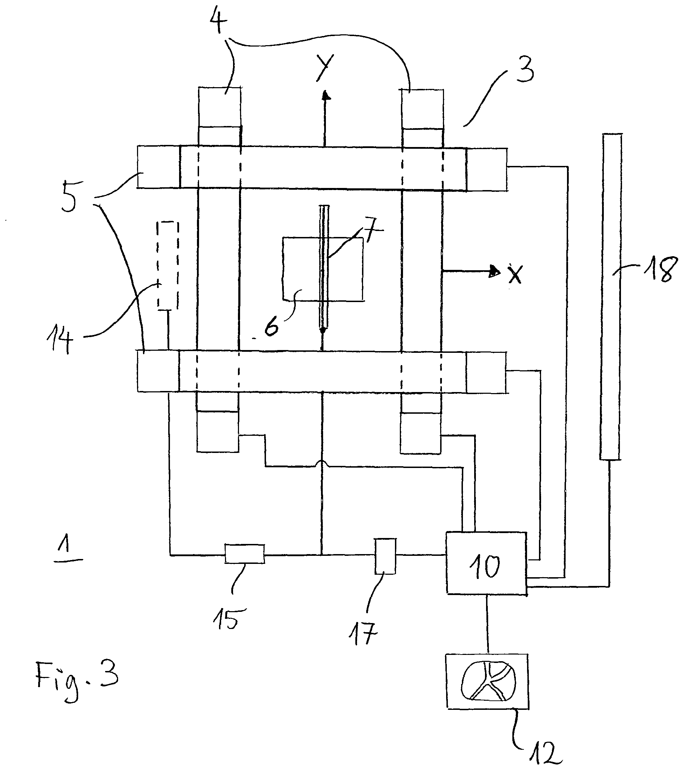

In Fig. 3 ist schematisch eine Vorrichtung 1 zur Bildgebung mittels magnetischer Kleinstpartikel dargestellt. Die Vorrichtung 1 umfasst hierzu einen Magnetfeldgenerator 3, der aus zwei orthogonal zueinander positionierten Helmholtzspulenpaaren 4 und 5 gebildet ist. Das Helmholtzspulenpaar 4 erzeugt hierbei ein homogenes Magnetfeld in x-Richtung. Das Helmholtzspulenpaar 5 erzeugt ein hierzu orthogonales homogenes Magnetfeld in γ-Richtung. Über eine sinusförmige Ansteuerung der entsprechenden Spulen lässt sich ein um die z-Achse rotierendes äußeres Magnetfeld erzeugen.

Im Innenraum zwischen den Helmholtzspulenpaaren 4,5 ist ein Messvolumen 6 gebildet, in dem beispielsweise eine räumliche Verteilung eines magnetischen Kleinstpartikel enthaltenden Kontrastmittels erfasst werden soll. Dazu rotiert das erzeugte äußere Magnetfeld mit einer entsprechend vorgegebenen Frequenz und einer entsprechend gewählten Feldstärke derart, dass die Einzelmagnetisierungen der Kleinstpartikel asynchron zum äußeren Feld rotieren. Die senkrecht zur z-Achse ausgerichtete superpositionierte Quermagnetisierung des im Messvolumen 6 befindlichen Partikelensembles wird über eine Messeinrichtung 7 induktiv erfasst. Die Messeinrichtung 7 ist eine parallel zur z-Achse ausgerichtete Solinoid-Spule.

Zur Ansteuerung der Helmholtzspulen 4,5 sowie zur Erfassung des von der Messeinrichtung 7 empfangenen Messsignals ist eine Steuervorrichtung 10 vorgesehen. Zusätzlich zu dem rotierenden Magnetfeld wird über eine entsprechende Ansteuerung der Helmholtzspulen 4,5 ein magnetisches Gradientenfeld erzeugt, welches zur Ortskodierung der Frequenzanteile der superpositionierten Quermagnetisierung dient. Alternativ können für alle drei Raumrichtungen Gradientenspulen zur Erzeugung insbesondere linearer Gradienten vorgesehen sein. Hierfür eignen sich in besonderem Maße so genannte Golay-Biplanarspulen.

Über die Steuervorrichtung 10 kann eine Abbildung der räumlichen Verteilung der magnetischen Kleinstpartikel oder eine Abbildung von Eigenschaften deren chemischer Umgebung durch entsprechende Ansteuerung der Magnetspulen erzeugt werden. Beispielsweise kann ein Refokussierungsmagnetfeld zur Phasenausrichtung der Einzelmagnetisierungen erzeugt und anschließend zu einer zweidimensionalen Bildgebung der Frequenzraum durch ein kartesisches Raster abgetastet werden, wozu durch Variation der Gradientenfelder jeweils eine Phasenkodierung vorgenommen wird.

Zu einer Unterdrückung von induktiven Spannungen oder Strömen, die von den äußeren Magnetfeldern herrühren, ist zwischen den Spulen des Spulenpaars 5 eine Gegenkopplungsaufnahme 14 angeordnet. Deren Ausgangssignal wird über eine Kopplungseinheit 15 (beispielsweise induktiv) dem Ausgangssignal der Messeinrichtung 7 gegengekoppelt. Weiter ist ein Tiefpassfilter 17 zur Selektion der gegenüber der Rotationsfrequenz des äußeren Magnetfeldes verringerten mittleren asynchronen Rotationsfrequenzen vorgesehen.

Die Steuervorrichtung 10 ist mit einer Abbildungsvorrichtung 12 verbunden, die beispielsweise als ein Informationsträger ausgebildet ist, der die ermittelte Abbildung anzeigt. Zur physiologischen Bildzuordnung ist eine Vorrichtung 18 zur Bildgebung mittels des Low-Field-Kernspinresonanzverfahrens kombiniert.

Bezugszeichenliste

| | |
|---|---|
| a) | mittlere Rotationsrate (kleines Feld) |
| b) | mittlere Rotationsrate (großes Feld) |
| $\tau$ | Querrelaxation |
| $M_Q$ | Quermagnetisierung |
| 1 | Vorrichtung |
| 3 | Magnetfeldgenerator |
| 4 | Helmholtzspulenpaar, x-Richtung |
| 5 | Helmholtzspulenpaar, y-Richtung |
| 6 | Messvolumen |
| 7 | Messeinrichtung |
| 10 | Steuervorrichtung |
| 12 | Abbildungsvorrichtung |
| 14 | Gegenkopplungsaufnahme |

15      Kopplungseinheit
17      Tiefpassfilter
18      Low-Field-Kernspinresonanz-Vorrichtung

**Patentansprüche**

1. Verfahren zur Bildgebung aus einer Verteilung magnetischer Kleinstpartikel, wobei

   - mittels eines um eine Längsachse (z) rotierenden äußeren Magnetfelds geeigneter Feldstärke und Rotationsfrequenz die Magnetisierungen der Kleinstpartikel in eine zum Magnetfeld asynchrone Rotation versetzt werden, wodurch sich für ein Partikelensemble eine von der Feldstärke abhängige asynchrone mittlere Rotationsfrequenz ergibt,
   - mittels eines magnetischen Gradientenfelds der asynchronen mittleren Rotationsfrequenz jeweiliger Partikelensemble eine Ortsabhängigkeit aufgeprägt wird,
   - die Frequenzanteile einer superpositionierten Quermagnetisierung ($M_Q$) des Partikelensembles erfasst werden und
   - mittels der Frequenzanteile eine ortsaufgelöste Verteilung der Quermagnetisierung ($M_Q$) der Kleinstpartikel ermittelt und ausgegeben wird.

2. Verfahren nach Anspruch 1, wobei zu einer Frequenzkodierung ein magnetisches Gradientenfeld während der Erfassung der Frequenzanteile der Quermagnetisierung ($M_Q$) erzeugt wird.

3. Verfahren nach Anspruch 1 oder 2, wobei zu einer Phasenkodierung, insbesondere vor der Erfassung der Frequenzanteile der Quermagnetisierung ($M_Q$), ein magnetisches Gradientenfeld für eine vorgegebene Zeitspanne erzeugt wird.

4. Verfahren nach einem der vorhergehenden Ansprüche, wobei ein magnetisches Gradientenfeld durch räumliche Variation der Feldstärke des rotierenden Magnetfeldes erzeugt wird.

5. Verfahren nach einem der vorhergehenden Ansprüche, wobei die Frequenzanteile der Quermagnetisierung ($M_Q$) mehrmals für verschiedene Gradientenfelder erfasst werden.

6. Verfahren nach einem der vorhergehenden Ansprüche,

   - wobei in einem ersten Schritt für eine Zeitspanne ein erstes Gradientenfeld entlang einer ersten Richtung erzeugt wird,
   - wobei in einem zweiten Schritt ein zweites Gradientenfeld entlang einer zweiten Richtung erzeugt wird,
   - wobei in einem dritten Schritt die Frequenzverteilung der Quermagnetisierung ($M_Q$) erfasst wird,
   - wobei in einem vierten Schritt die Schritte eins bis drei mit einer Anzahl abgeänderter erster Gradientenfelder wiederholt werden, und
   - wobei aus dem gewonnenen Satz an Messsignalen eine zweidimensionale Verteilung der Quermagnetisierung ($M_Q$) ermittelt und ausgegeben wird.

7. Verfahren nach Anspruch 6,

   - wobei nach dem zweiten Schritt ein drittes Gradientenfeld entlang einer dritten Richtung erzeugt wird,
   - wobei die Schritte eins bis vier mit einer Anzahl abgeänderter zweiter Gradientenfelder wiederholt werden und
   - wobei aus dem Satz an gewonnenen Messsignalen eine dreidimensionale Verteilung der Quermagnetisierung ($M_Q$) ermittelt und ausgegeben wird.

8. Verfahren nach einem der vorhergehenden Ansprüche, wobei vor Messung mittels eines kurzzeitigen magnetischen Refokusierungsfelds die Magnetisierungen der Kleinstpartikel in eine zum rotierenden Magnetfeld synchrone Rotation versetzt werden.

9. Verfahren nach einem der vorhergehenden Ansprüche, wobei zu einer Phasenkodierung ein rotierendes magnetisches Gradientenfeld mit seinem Gradientenverlauf räumlich über das Meßvolumen geschoben wird, wobei die Feldstärke im Gradientenverlauf über einen Wert, der zur Anregung einer asynchronen Rotation der Kleinstpartikel führt, bis wenigstens zu einem Wert, der zur Anregung einer synchronen Rotation der Kleinstpartikel führt, ansteigt.

10. Verfahren nach Anspruch 9, wobei zur Bildgebung die Phasenkodierung mehrfach mit veränderter Verschiebungsrate wiederholt wird.

11. Verfahren nach einem der vorhergehenden Ansprüche, wobei die rotierenden Magnetisierungen der Kleinstpartikel durch eine Drehrichtungsumkehr des rotierenden Magnetfelds in Phase gebracht werden.

12. Verfahren nach einem der vorhergehenden Ansprüche,
wobei mittels des oder jedes Gradientenfeldes eine lineare Ortsabhängigkeit der mittleren Rotationsfrequenz erzeugt wird.

13. Verfahren nach einem der vorhergehenden Ansprüche,
wobei zugleich eine Bildgebung mittels eines Low-Field-Kernspinresonanz-Verfahrens zu einer Bildzuordnung durchgeführt wird.

14. Vorrichtung (1) zur Bildgebung mittels magnetischer Kleinstpartikel, mit einem Magnetfeldgenerator (3) eingerichtet zur Erzeugung eines magnetischen Gradientenfeldes und eines um eine Längsachse (z) rotierenden Magnetfeldes, mit einer Messeinrichtung (7) eingerichtet zur Erfassung der Frequenzanteile einer superpositionierten Quermagnetisierung ($M_Q$) eines Partikelensembles, mit einer Steuervorrichtung (10), die eingerichtet ist, den Magnetfeldgenerator (3) entsprechend einem Verfahren nach einem der vorhergehenden Ansprüche anzusteuern und die erfassten Frequenzanteile in eine ortsaufgelöste Verteilung der Quermagnetisierung ($M_Q$) der Kleinstpartikel umzurechnen, und mit einer Ausgabevorrichtung (12) eingerichtet zur Ausgabe der gewonnenen Verteilung.

15. Vorrichtung (1) nach Anspruch 14,
wobei die Messeinrichtung (7) eine Anzahl von parallel zur Längsachse (z) ausgerichteter Induktionsspulen umfasst.

16. Vorrichtung (1) nach Anspruch 15,
wobei das Eingangssignal des Magnetfeldgenerators (3) mit dem Spulenausgangssignal gegenphasig gekoppelt ist.

17. Vorrichtung (1) nach einem der Ansprüche 14 bis 16, kombiniert mit einer hierzu relativ positionierten Vorrichtung (14) zur Durchführung eines Low-Field-Kernspinresonanzverfahren.

**Claims**

1. A method for imaging from a distribution of magnetic small particles, the method comprising:

   - setting the magnetizations of the small particles in a rotation which is asynchronous with respect to the magnetic field using an external magnetic field of suitable field strength and rotation frequency rotating about a longitudinal axis (z), wherein an asynchronous average rotation frequency dependent on the field strength results for a particle ensemble,
   - applying a magnetic gradient field to impose a position dependency on the asynchronous average rotation frequency of the respective particle ensemble,
   - recording the frequency components of a superimposed transverse magnetization ($M_Q$) of the particle ensemble and
   - determining a spatially resolved distribution of the transverse magnetization ($M_Q$) of the small particles using the frequency components and outputting the spatially resolved distribution.

2. The method as claimed in claim 1, wherein a magnetic gradient field is generated for frequency encoding during the recording of the frequency components of the transverse magnetization ($M_Q$).

3. The method as claimed in claim 1 or 2, wherein a magnetic gradient field is generated for a predetermined time period for phase encoding, in particular before recording the frequency components of the transverse magnetization ($M_Q$).

4. The method as claimed in one of the preceding claims, wherein a magnetic gradient field is generated by spatial variation of the field strength of the rotating magnetic field.

5. The method as claimed in one of the preceding claims, wherein the frequency components of the transverse magnetization ($M_Q$) are recorded several times for different gradient fields.

6. The method as claimed in one of the preceding claims,

   - wherein a first gradient field is generated along a first direction in a first step for a time period,
   - wherein a second gradient field is generated along a second direction in a second step,
   - wherein the frequency distribution of the transverse magnetization ($M_Q$) is recorded in a third step,
   - wherein steps one to three are repeated with a number of modified first gradient fields in a fourth step, and
   - wherein a two-dimensional distribution of the transverse magnetization ($M_Q$) is determined from the set of measurement signals obtained and is output.

7. The method as claimed in claim 6,

   - wherein a third gradient field is generated along a third direction after the second step,
   - wherein steps one to four are repeated with a number of modified second gradient fields, and
   - wherein a three-dimensional distribution of the transverse magnetization ($M_Q$) is determined from the set of measurement signals obtained and is output.

8. The method as claimed in one of the preceding claims, wherein the magnetizations of the small particles are set in a rotation synchronous with the rotating magnetic field before the measurement using a short-term magnetic refocusing field.

9. The method as claimed in one of the preceding claims, wherein a rotating magnetic gradient field with its gradient profile is spatially shifted over the measurement volume for a phase encoding, wherein the field strength in the gradient profile increases beyond a value which leads to excitation of an asynchronous rotation of the small particles, at least up to a value which leads to the excitation of a synchronous rotation of the small particles.

10. The method as claimed in claim 9, wherein the phase encoding is repeated several times with a modified displacement rate for the imaging.

11. The method as claimed in one of the preceding claims, wherein the rotating magnetizations of the small particles are brought into phase by a rotation direction reversal of the rotating magnetic field.

12. The method as claimed in one of the preceding claims, wherein a linear position dependency of the average rotation frequency is generated using the or each gradient field.

13. The method as claimed in one of the preceding claims, wherein imaging using a low-field nuclear magnetic resonance method is simultaneously carried out for image allocation.

14. A device (1) for imaging using magnetic small particles, comprising a magnetic field generator (3) configured to generate a magnetic gradient field and a magnetic field rotating about a longitudinal axis (z), comprising a measuring instrument (7) configured to record the frequency components of a superimposed transverse magnetization ($M_Q$) of a particle ensemble, comprising a control device (10) configured to drive the magnetic field generator (3) corresponding to a method as claimed in one of the preceding claims and to convert the recorded frequency components into a spatially resolved distribution of the transverse magnetization ($M_Q$) of the small particles, and comprising an output device (12) configured to output the distribution obtained.

15. The device (1) as claimed in claim 14, wherein the measuring instrument (7) comprises a number of induction coils aligned parallel with the longitudinal axis (z).

16. The device (1) as claimed in claim 15, wherein the input signal of the magnetic field generator (3) is coupled in antiphase with the coil output signal.

17. The device (1) as claimed in one of claims 14 to 16, combined with a device (14) positioned relative thereto for carrying out a low-field nuclear magnetic resonance method.

**Revendications**

1. Procédé d'imagerie à partir d'une distribution de petites particules, dans lequel

 - les magnétisations des petites particules sont mises en rotation asynchrone par rapport au champ magnétique au moyen d'un champ magnétique externe d'intensité de champ et de fréquence de rotation appropriées tournant autour d'un axe longitudinal (z), cela entraînant pour un ensemble de particules une fréquence de rotation moyenne asynchrone dépendant de l'intensité du champ,
 - une dépendance locale est conférée à l'ensemble de particules respectif au moyen d'un champ de gradient magnétique ayant la fréquence de rotation moyenne asynchrone,
 - les composantes de fréquence d'une magnétisation transversale ($M_Q$) superposée de l'ensemble de particules sont détectées et
 - une distribution spatialement résolue de la magnétisation transversale ($M_Q$) des petites particules est déterminée au moyen des composantes de fréquences et est fournie en sortie.

2. Procédé selon la revendication 1, dans lequel, pour un codage de fréquence, un champ de gradient magnétique est généré pendant la détection des composantes de fréquence de la magnétisation transversale ($M_Q$).

3. Procédé selon la revendication 1 ou 2, dans lequel, pour un codage de phase, en particulier avant la détection des composantes de fréquence de la magnétisation transversale ($M_Q$), un champ de gradient magnétique est généré au cours d'un intervalle de temps prédéterminé.

4. Procédé selon l'une des revendications précédentes, dans lequel un champ de gradient magnétique est généré par une variation spatiale de l'intensité de champ du champ magnétique tournant.

5. Procédé selon l'une des revendications précédentes, dans lequel les composantes de fréquence de la magnétisation transversale ($M_Q$) sont détectées plusieurs fois pour différents gradients de champ.

6. Procédé selon l'une des revendications précédentes,

- dans lequel, lors d'une première étape au cours d'un intervalle de temps, un premier champ de gradient est généré le long d'une première direction,
- dans lequel, lors d'une deuxième étape, un deuxième gradient de champ est généré le long d'une deuxième direction,
- dans lequel, lors d'une troisième étape, la distribution de fréquence de la magnétisation transversale ($M_Q$) est détectée,
- dans lequel, lors d'une quatrième étape, les étapes 1 à 3 dont répétées avec un certain nombre de premiers champs de gradient modifiés, et
- dans lequel une distribution bidimensionnelle de la magnétisation transversale ($M_Q$) est déterminée à partir de l'ensemble obtenu de signaux de mesure et est fournie en sortie.

7. Procédé selon la revendication 6,

- dans lequel, après la deuxième étape, un troisième champ de gradient est généré le long d'une troisième direction,
- dans lequel les étapes 1 à 4 sont répétées avec un certain nombre de deuxièmes champs de gradient modifiés, et
- dans lequel une distribution tridimensionnelle de la magnétisation transversale ($M_Q$) est déterminée à partir de l'ensemble de signaux de mesure obtenus et est fournie en sortie.

8. Procédé selon l'une des revendications précédentes,
dans lequel, avant la mesure effectuée au moyen d'un champ de refocalisation magnétique de courte durée, les magnétisations des petites particules sont mises en rotation synchrone par rapport au champ magnétique tournant.

9. Procédé selon l'une des revendications précédentes,
dans lequel, pour un codage de phase, un champ de gradient magnétique tournant est soumis à un déplacement spatial à travers le volume de mesure avec sa courbe de variation de gradient, dans lequel la courbe de variation du gradient d'intensité de champ croît au-dessus d'une valeur qui conduit à la stimulation d'une rotation asynchrone des petites particules, jusqu'à au moins une valeur qui conduit à la stimulation d'une rotation synchrone des petites particules.

10. Procédé selon la revendication 9,
dans lequel le codage de phase est répété plusieurs fois à des fins de formation d'image avec des vitesses de déplacement modifiées.

11. Procédé selon l'une des revendications précédentes,
dans lequel les magnétisations tournantes des petites particules sont mises en phase par une inversion du sens de rotation du champ magnétique tournant.

12. Procédé selon l'une des revendications précédentes,
dans lequel une dépendance locale linéaire de la fréquence de rotation moyenne est générée au moyen du ou de chaque champ de gradient.

13. Procédé selon l'une des revendications précédentes,
dans lequel une formation d'image est effectuée simultanément au moyen d'un procédé de résonance magnétique en faible champ afin de réaliser une association d'image.

14. Dispositif (1) de formation d'image au moyen de petites particules magnétiques, comportant un générateur de champ magnétique (3) conçu pour générer un champ de gradient magnétique et un champ magnétique tournant autour d'un axe longitudinal (z), comportant un dispositif de mesure (7) conçu pour détecter les composantes de fréquence d'une magnétisation transversale ($M_Q$) superposée d'un ensemble de particules, comportant un dispositif de commande (10) qui est conçu pour commander le générateur de champ magnétique (3) d'une manière qui correspond à un procédé selon l'une des revendications précédentes et pour convertir les composantes de fréquence détectées en une distribution spatialement résolue de la magnétisation transversale ($M_Q$) des petites particules, et comportant un dispositif de sortie (12) conçu pour fournir en sortie la distribution obtenue.

15. Dispositif (1) selon la revendication 14,
dans lequel le dispositif de mesure (7) comprend un certain nombre de bobines d'induction orientées parallèlement à l'axe longitudinal (z).

16. Dispositif (1) selon la revendication 15,
dans lequel le signal d'entrée du générateur de champ magnétique (3) est couplé en opposition de phase au signal de sortie de bobine.

17. Dispositif (1) selon l'une des revendications 14 à 16, combiné à un dispositif (14) positionné par rapport à celui-ci pour mettre en oeuvre un procédé de résonance magnétique à faible champ.

Fig. 1

Fig. 2

Fig. 3

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- WO 2004091390 A2
- US 20080220411 A1

**In der Beschreibung aufgeführte Nicht-Patentliteratur**

- **BERNHARD GLEICH ; JÜRGEN WEIZENECKER.** Tomographic Imaging Using the Non-Linear Response of Magnetic Particles. *Nature,* 30. Juni 2005, vol. 435